# EUROPEAN PATENT SPECIFICATION

(11) **EP 2 991 642 B1**
(45) Date of publication and mention of the grant of the patent: **03.07.2019**
(21) Application number: 14721334.2
(22) Date of filing: 29.04.2014
(51) Int. Cl.: A61K 31/327, A61K 33/18, A61P 31/00, A61K 9/00, A61K 33/40, A61K 31/375, A61K 31/573

(54) **ANTIMICROBIAL COMPOSITIONS FOR USE IN THE TREATMENT OF MASTITIS IN RUMINANTS**
ANTIMIKROBIELLE ZUSAMMENSETZUNGEN ZUR BEHANDLUNG VON MASTITIS IN WIEDERKÄUERN
COMPOSITIONS ANTIMICROBIENNES DESTINÉES AU TRAITEMENT DE LA MASTITE DES RUMINANTS

(30) Priority: 01.05.2013 EP 13166137
(43) Date of publication of application: 09.03.2016
(73) Proprietor: National University of Ireland Galway, Galway (IE)
(72) Inventor: O'FLAHERTY, Vincent, Moycullen Co. Galway (IE); McCAY, Paul, Newcastle Co. Galway (IE)
(74) Representative: Carpmaels & Ransford LLP
(86) International application number: PCT/EP2014/058766
(87) International publication number: WO 2014/177590

(56) References cited:
- WO-A1-94/09635
- US-A- 4 567 036
- US-A- 4 935 248
- US-A- 5 066 497
- US-A- 5 639 481
- US-A1- 2007 286 907
- US-A1- 2007 292 360
- SIMONE ULIVIERI ET AL: "Prevention of post-operative infections in spine surgery by wound irrigation with a solution of povidoneâ iodine and hydrogen peroxide", ARCHIVES OF ORTHOPAEDIC AND TRAUMA SURGERY ; INCLUDING ARTHROSCOPY AND SPORTS MEDICINE, SPRINGER, BERLIN, DE, vol. 131, no. 9, 22 January 2011 (2011-01-22), pages 1203-1206, XP019941751, ISSN: 1434-3916, DOI: 10.1007/S00402-011-1262-0
- LI HSIU-PING ET AL: "Bacterial Production of Organic Acids Enhances H2O2-Dependent Iodide Oxidation", ENVIRONMENTAL SCIENCE AND TECHNOLOGY, AMERICAN CHEMICAL SOCIETY. EASTON, PA, US, vol. 46, no. 9, 1 May 2012 (2012-05-01), pages 4837-4844, XP009171065, ISSN: 0013-936X
- Seymour J Klebanoff: "Iodination of Bacteria: A bactericidal mechanism", , 1 January 1967 (1967-01-01), pages 1063-1079, XP055070609, Retrieved from the Internet: URL:http://jem.rupress.org/content/126/6/1 063.full.pdf#page=1&view=FitH [retrieved on 2013-07-10]
- Seymour J Klebanoff: "Prooxidant activity of transferrin and lactoferrin", , 1 November 1990 (1990-11-01), XP055071227, Retrieved from the Internet: URL:http://jem.rupress.org/content/172/5/1 293.full.pdf [retrieved on 2013-07-15]

## Description

### Field of Invention

This invention relates to compositions as defined herein for use in methods for treatment or prevention of mastitis in ruminants. The composition is administered into the udder of an animal as a highly effective treatment for mastitis, or as a prophylactic therapy, by means of an intra-mammary infusion. The milk produced by the animal, during treatment using the composition and method of the invention, is free of residues, such as antibiotics, antimicrobial agents or antimicrobial proteins, which could affect its suitability for drinking or in the production of milk products, such as cheese or yoghurt. The compositions and methods disclosed are also useful in treating and preventing lung infections; and infections in burns and wounds; and other infections caused by biofilms. The disclosed compositions may also be used on medical devices to prevent infection.

### Background to the invention

Bovine mastitis is the costliest medical condition in veterinary husbandry. Mastitis occurs as a result of a bacterial infection in the udder. There are three elements involved in such infections; microorganisms, environmental factors, and the cow itself. *Staphylococci* and *Streptococci* are the cause of c. 90% of infections though others, such as *E. coli* or pseudomonads can also cause infection. Nutrition, bedding and milking technique all influence the likelihood of infection. Moreover, the stage of lactation will have an effect (teats can crack and become damaged as the lactation cycle progresses) and the number of lactations undergone by an animal will dictate the likelihood of inflammation (though not necessarily infection). Infection of the udder will lead to a decreased yield in milk production, and an increase of the Somatic Cell Count (SCC) in milk. SCC levels are used by dairy farmers and milk processers as a proxy measure for milk quality and the presence of bacterial infection. An SCC of >800,000 per ml, coupled with signs of discomfort (such as swelling, tenderness of the udder) in an animal, would be considered a clinical case of mastitis. In sub-clinical cases, there may be no outward signs of infection in the animal but an elevated SCC Count in milk. Farmers are often paid for their milk based on the SCC counts, with higher quality milk, classified based on low SCC levels, attracting a premium.

The typical treatment for mastitis involves the use of antibiotic therapies. The use of antibiotics often precludes the sale of the animal's milk during and post treatment, for a period of up to 9 days. The requirement to discard, or at least not sell, milk during antibiotic treatment is a significant additional economic cost to the milk producer, associated with mastitis. The no-sale requirement is put in place in order to:
**(i) Prevent antibiotic residues from entering the human food chain:** Traditional antibiotic therapies all require a milk discard during and post-treatment for a number of days. This is to ensure that there are no antibiotics in the food chain. Antibiotics in the food chain will have two important effects - they increase the possibility of bacterial resistance to the drug by a continued contact with the drug. Cephalasporins (following an earlier ban of fluoroquinolone use), for example, have recently been banned for husbandry use in the US because they are such an important class of drug for human pathogen therapeutics (Federal Register /Vol. 77, No. 4 / Friday, January 6, 2012). Secondly, the presence of antibiotics can greatly affect the flora of the human gut, inhibiting important, pro-biotic bacteria, and allowing the proliferation of potential pathogenic bacteria that normally do not gain a 'foot-hold' in such an environment.
**(ii) Ensure bacterial starter cultures for cheese and yogurt production are not inhibited:** The presence of antibiotic residues will inhibit the starter cultures (such as lactobacilli) used for the manufacturing of both cheeses and yogurts. These cultures are typically composed of organisms very sensitive to antibiotics, and thus a long milk discard period is required following antibiotic usage, to ensure that the starter cultures are not inhibited. The use of a Delvo test at the dairy level is a relatively simple method for screening for antimicrobial residues in milk. It is based on the use of a heat-tolerant microorganism that is sensitive to antimicrobials, and is considered the 'Gold Standard' in dairy practices. Inhibition of the organism indicates the presence of antimicrobials in the milk. Growth of the microorganism indicates that there are no antimicrobials present. Antimicrobial-containing milk is discarded, at a large cost to the farmer, as it is not suitable for use in post-processing. Various antibiotics are presently sold and all require a milk discard of a certain length. For example, Tetra Delta requires a milk discard for the two days during treatment and for 5 days post treatment. Ubro Yellow requires a milk discard for the three days during treatment and for 6 days post treatment. Terrexine requires a milk discard for the one day of treatment and for 6 days post treatment. No antibiotics are sold that would not require a milk discard. Milk discard is viewed by dairy farmers as the major cost incurred by a case of mastitis.

The use of compounds, other than antibiotics, to treat mastitis, without a withdrawal period, has been proposed. United States Patent No. 6794181 describes the isolation and use of a lantibiotic-classed compound named nisin. The compound has antimicrobial properties. Nisin, and similar compounds (e.g., US Patents 07/317,627; 5950269 and 20110269671) are antimicrobial peptides, rather than antibiotics, and thus no antibiotic residues would be present in milk. In the absence of other preservation methods, nisin does not, however, inhibit Gram-negative bacteria, yeasts, or moulds. Consequently, nisin is mainly used as a food preservative and not as a therapeutic, in combination with other synergistic food preservation methods such as low pH and high salt concentrations. Moreover, nisin residues in milk may also inhibit starter cultures for cheese and yogurt production and thus interfere with downstream processing of milk in a manner similar to antibiotics. It has been found, for example, that nisin residues in milk could lead to some interference with cultured dairy products (certain cheeses, yogurts) if a high proportion of animals are treated at any one time. Nisin use was also shown to elevate the somatic cell count of the animals during treatment, a serious drawback when payment to the farmer is based on SCC levels. The acquisition of resistance to nisin and similar molecules by pathogenic bacteria has also been reported.

In addition to concerns about the development of antibiotic resistance with respect to human medicine, a major problem in dairy husbandry is antimicrobial resistance in animals, associated with historical antibiotic usage. Microbial resistance to antibiotic drugs will become prevalent upon repeated usage, such that any particular antibiotic will become completely ineffective as a therapy at some point - this is widely recognised as a critical and urgent challenge in both human and veterinary medicine. Causative organisms for mastitis include the bacteria *Escherichia coli, Staphylococcus aureus, Streptococcus dysgalactiae, agalactiae, Streptococcus uberis,* and *Pseudomonas aeruginosa.* Of these, *S. aureus* is a particular problem because of the phenotype of the microorganism. The bacterial strain is capable of infecting the epithelial lining of the udder, thus evading the antibiotic. Moreover, the organism is generally found growing as a biofilm. In the biofilm phenotype, microorganisms are much more resilient to the actions of antimicrobials. As such, they are much more difficult to treat. The antibiotic treatment of *S*. *aureus* infections in cows, for example, often involves two phases. Firstly, the antibiotics will cause an initial decrease in SCC, indicating the killing of the bacteria. However, the antibiotic will not access the cells in the epithelial lining. Within a short time frame (for example, 2-3 weeks) the infection will re-establish itself as bacterial cells growing in the epithelial lining are shed in the udder once more to proliferate in the nutrient rich environment. This type of case would now be classed as 'chronic mastitis'. Treatment of such cases is particularly difficult, as repeated use of the antibiotic results in a bacterial strain that, due to repeated contact with the drug, is highly likely to develop resistance characteristics. Treatment of these types of cases has a poor chance of success, even using the most potent of current antibiotic therapies. At this stage, it will normally be economically unfeasible to keep the animal, and thus the animal would be culled and the farmer would replace the animal at a cost of >€1,000 ($1,300)

The economic costs associated with the occurrence of mastitis thus include: reduction of milk yield, loss of income due to poorer quality milk produced, veterinarian charge, antibiotic prescription, loss of income due to withholding of milk and replacement of culled animals.

### Microbial Infections

A further object of the disclosure is the prevention and treatment of a number of other bacterial infections. Drug delivery and resistance to antibiotics is a major problem in the treatment of cystic fibrosis (CF), tuberculosis (TB) and pnuemonias. Antibiotic resistance often occurs as a result of only sub-inhibitory concentrations of the drug reaching the target site, i.e. lungs. Chronic infections will often result from this situation, seriously impairing the health of the patient. Addition of a composition of iodide and hydrogen peroxide could act prophylactically on people diagnosed with CF, or could be used to treat patients that have already developed lung infections. Such a composition, delivered as a nebulised spray, or as a physiological saline composition (or indeed using water as a carrier) has the potential to be used in hospitals to minimise and control infections, especially those caused by antibiotic resistant strains of bacteria. This is a particular risk during surgical procedures, where body cavities are open to the environment.

### Burns/Skin/Mouth

Other infections that are suitable targets for the compositions and methods of the disclosure are those incurred as a result of burns and open, or chronic, wounds. The advantage of using the compositions, treatments or methods over present antibiotic treatment regimens would be similar to those described above in treating CF or TB regarding safety, efficacy, and low risk of bacterial resistance to the treatment. The present inventors have shown that the described compositions are effective in the treatment of biofilm based cells (those attached to a solid stratum). This would be the bacterial phenotype most noted in the lungs of TB/CF patients, and in open wounds or burns. The biofilm phenotype confers a generalised tolerance to the bacteria against a wide range of antibiotics.

The composition of the disclosure could also serve as a general antibacterial solution, having numerous purposes. For example, a mouthwash containing the antibacterial composition could help prevent the formation of biofilms within the mouth. Likewise, an antibacterial nasal rinse could be used to help alleviate sinus problems, such as sinusitis or allergic rhinitis. Currently, steroids and saline nasal washes are used. An antibacterial saline wash would, however, also help to prevent and combat bacterial colonisation of the nasal cavity.

### Medical Devices

The use of the antimicrobial composition of the disclosure would also hold a number of advantages in the treatment and prevention of bacterial infections on an implanted medical device. Infections (in the form of a recalcitrant biofilm) are extremely common in various devices such as catheters.

### Antifungal agent

Infections can occur as a result of yeast or fungal growth as well as bacteria. As such, a therapeutic regime capable of exerting antimicrobial activity (as opposed to antibacterial activity, as is the case with antibiotics) would be of great benefit. To this end, the described examples of compositions are capable of eradicating a number of fungal strains including, but not limited to: *Candida* (strains of fungus are typically described as opportunistic pathogens. They can cause a variety of skin conditions, as well as vulvovaginitis and urinary tract infections); *Saccharomyces cerevesiae* (commonly known as bakers' yeast) is an important organism in food production. It also poses great problems for the drinks industry, and is a typical organism found on beer lines, and is the cause of beverage spoilage.

The disclosure also finds use as an anti-viral agent.

Hydrogen peroxide is used as a high level disinfectant of surfaces, either alone in aqueous solution, or in combination with other chemicals. A 3% solution is also used as an antiseptic. These applications are unsuitable for use in an antimicrobial therapy within the body of a human or an animal, due to the damage caused by elevated concentrations (0.15% upwards) of peroxide to mammalian tissue. Furthermore, elevated concentrations of hydrogen peroxide have been shown to impede healing and lead to scarring of damaged tissue (in, for example wounds and burns) because it destroys newly formed cells.

Hypoiodite (IO⁻) has been discussed in terms of being a by-product in processes such as UV irradiation and as a product of the lactoperoxidase (or other peroxidase) system (EP 2510944 A1, Patent No. US 2012/0021071 A1, Patent No. US 2012/0128650 A1, Patent No. 5607681).

Hypoiodite (IO⁻), produced as a result of the reaction between peroxide, a peroxidase enzyme and iodide can be bacteriocidal to Gram-negative microorganisms when they are grown in laboratory media, but the literature teaches that this approach will be ineffective under physiological conditions as the production of IO⁻ is inhibited by the presence of thiocyanate, at concentrations of the latter compound normally found in saliva, milk and other physiological settings (Klebanoff et al., 1967, J Exp Med 1967 126(6):1063-78; Tenovuo et al. 2002 Oral Diseases 8, 23-29). IO⁻ is generally regarded as being bacteriostatic (i.e. suppresses the growth only) to Gram-positive organisms as part of the natural antimicrobial lactoperoxidase system. *E. coli* and *P. aeruginosa* are Gram-negative organisms, while *S. aureus* and the *Streptococci* are Gram-positive organisms.

Hydrogen peroxide itself is antimicrobial at high concentrations, but it is mainly effective against gram positive bacteria and is regarded as being only bacteriostatic to gram negative bacteria, such as *P. aeruginosa.* Moreover, the presence of catalase in bacteria makes solutions of hydrogen peroxide at a concentration below 3% less effective, even against gram positives . In a similar fashion, catalases present in tissues can render hydrogen peroxide still less antimicrobial *in vivo.* For example, in a clinical study on *S. aureus* in blister wounds no reduction in bacterial load was achieved using 3% hydrogen peroxide. A review of the use of hydrogen peroxide in a variety of studies on wounds found that "In conclusion, hydrogen peroxide appears not to negatively influence wound healing, but it is also ineffective in reducing the bacterial count" (Drosou et al., 2003, Wounds; 15(5). The prior art thus teaches that use of compositions containing less than 3% hydrogen peroxide for bacteriocidal purposes would be ineffective for *in vivo* wound applications. Similar findings are reported with respect to physiological fluids. For example, no peroxide-related antimicrobial activity was found either when hydrogen peroxide was infused into patients infected with a variety of organisms. There was no bactericidal activity when hydrogen peroxide was infused into blood of rabbits infected with peroxide-sensitive *E. coli.* Moreover, increasing the concentration of peroxide *ex-vivo* in rabbit or human blood containing *E.coli* produced no evidence of bactericidal activity. The lack of an antimicrobial effect of high concentrations of hydrogen peroxide was directly attributed to the presence of the peroxide-destroying enzyme, catalase.

Many pathogenic bacteria, including *S. aureus* and *E. coli,* express catalase enzymes that inactivate peroxide. The basis for designing a peroxidase enzyme system as an antimicrobial for use in food preservation, dentifrices, etc, therefore, is that the peroxidase enzyme is required to compete with the bacteria to convert sub-toxic concentrations of hydrogen peroxide (or other form of peroxide, such as sodium peroxide) into an antimicrobial agent before the peroxide is removed by the bacteria.

The literature teaches the inherent importance of the use of a peroxidase enzyme to catalyse the reaction between iodide and peroxide, at concentrations of the latter molecule that will not be toxic to mammals. Thus, the peroxidase enzyme (as well as the substrate and peroxide) has been thought of as essential to allow the natural antimicrobial system to work - "In order to be efficient, the three elements which make up the system need to present simultaneously" - Poutrel et al. (Ann. Rech Vet, 1982, 13 (1), 85-89). Magnusson et al. (J of Biological Chemistry, Vol. 259, No. 22, 1984) describe, in detail, the central role of lactoperoxidase in transferring an oxygen molecule from the available peroxide to an available iodide molecule. They teach that a peroxidase enzyme is essential to regulate the reactions and that it had a large effect on the production of not only the major product IO⁻ , but IOH (hypoiodous acid), I₂ (iodine) and other iodous compounds that will also be produced as a result of the peroxide and iodide reaction.

Iodine has long been used as a method to kill bacteria. The most common found version is that of polyvinylpyrrilidone, PVP-I, also known as povidone-iodine. It is complexed to aid its stability as iodine (I₂) will quickly dissipate otherwise. It possesses the distinctive reddish brown colouration, and is often used as a topcial antiseptic during surgeries, or as a teat dip on dairy farms, minimising chances of mastitis infections transferring. Its limitations are well characterised as it reacts poorly with organic material, and can be toxic if in the blood stream.

IO⁻ has a relatively short half-life of a few hours, suggesting that it may exert only a transitory static effect in therapeutic treatments. This is supported by prior art reports describing: (i) poor effectiveness in killing Gram-positive organisms ('Due to mainly bacteriostatic effect of the system it is not possible to disguise poor milk quality', Guidelines for the Preservations of Raw Milk by the use of the Lactoperoxidase System, CAC/GL 13-1991, WHO "Lactoperoxidase System of Raw Milk Preservation - Call for data, 2005; Reiter and Harnulv 1984); (ii) transitory bacteriostatic effects wherein the bacteria once more start to proliferate after a short delay (Ishido et al., 2011 Milchwissenschaft 66 (1) 2011; Thomas et al., 1994, Infection and Immunity, Vol 62, No.2 p 529-535; Marks et al., 2001 J Appl. Micro. 91, 735-741; Kamau et al., 1990 Appl and Env. Micro. Vol. 56, No. 9; McLay et al., 2002, Int Jour of Food micro, 73, 1-9); and (iii) inability to eradicate bacteria growing in biofilms (Dufour et al., Journal of Food Protection, 67 (2004), pp 1438-1443; Abbeele et al., 1996, Int Rech Sci Stomotol Odontol 39 (1-2):57-61). This latter information teaches against the use of IO⁻ as a treatment in bovine mastitis, where biofilm-phenotypes occur regularly. One would expect that the IO⁻ compound would not be effective at completely killing the pathogens and curing a mastitis infection, based on published data.

The prior art, however, is incorrect with respect to the products of the reaction between low (<0.5%) concentrations of hydrogen peroxide and iodide, when this reaction takes place in the absence of a peroxidase enzyme. The present inventors have shown that these reaction products are effective at killing a wide-range of Gram-negative and Gram-positive organisms, including those growing in biofilm mode, and that they provide a highly effective therapy for mastitis, which allows the milk produced during treatment to be safely used for cheese production and other downstream processing. Moreover, we disclose that the presence of lactoperoxidase (or any other peroxidase or any other synergistic agent or compound) is not necessary to generate the antimicrobial activity in milk or other media; and the addition of a peroxidase enzyme did not enhance the antimicrobial efficacy of the products produced, nor their active half-life in milk or other media (Result 3). The short-lived nature of the antimicrobial activity, rather than being a hindrance, acts as a key advantage in the present invention. This is because we disclose a method to titre the reaction such that there is no antimicrobial activity remaining within milk when taken from the animal 8-12 hours after treatment. This is important, as bacterial starter cultures are used in the production of cheeses and yogurts, and would be inhibited if there were antimicrobial residues or agents present at that point.

Hypothiocyanite (OSCN⁻) is produced by a reaction of thiocyanate and peroxide, which is catalysed by a peroxidase enzyme. Numerous studies have examined the use of OSCN⁻ as a method of limiting bacterial growth. In addition, a number of patents have described the use of thiocyanate in a peroxidase reaction (EP 2510944 A1, Patent No. US 2012/0021071 A1, Patent No. US 2012/0128650 A1, Patent No. 5607681). All the described literature teach the use of a supplemented peroxidase enzyme to allow the reaction to proceed. Ishido *et al.,* 2011 described an OSCN⁻ method of inhibiting bacteria. The enzyme-catalysed system was incapable of eradicating *Pseduomonas* strains and *E. coli* strains, even with a sustained contact time of 48 hours. Other laboratory studies, based on OSCN⁻, have described bacteriostatic effects, rather than complete elimination of the bacteria, often with a minimal reduction in proliferation time (4 hours or less) in a majority of their isolates; or no inhibitory effects at all, particularly in mastitis causing pathogens such as *E. col.* Others have described the necessary use of immobilized enzymes to produce OSCN⁻, which is a configuration unsuited for use in a mastitis therapy. Carlsson and co-workers (Infect. Immunity, 44:581-586, 1984) further demonstrated that a byproduct of the reaction, SCN⁻, could potentiate the cytotoxic effects of the peroxide molecule under specific conditions, such as the presence of excess peroxide. These findings teach against the use of a OSCN⁻ model as a mastitis therapy for reasons of overall efficacy.

Despite this, the use of an OSCN⁻ model was described as a potential treatment of cystic fibrosis (Patent No. US 2012/0021071 A1), although no data on animal or human trials were included in that application. In US 2012/0021071 A1, however, the applicants acknowledge the need for additional extraneous compounds to enable biofilm removal as OSCN⁻ itself was insufficient for this purpose. A similar requirement for additional substances is described in US 8263138 B2. Indeed, the results presented in Table 2 of the present application also teach against use of OSCN⁻ in trying to kill biofilm cultures in the absence of synergistic compounds.

Surprisingly in the present invention, a composition containing low (<0.5%) concentrations of hydrogen peroxide and iodide - in the absence of a peroxidase enzyme - is sufficient to kill biofilm adhered cells, with no requirement for any synergistic compounds to be added to aid biofilm removal.

There are a number of other uses of the anti-microbial composition of the present disclosure. These include infections of the mammalian lung. Cystic fibrosis and tuberculosis are two diseases that are, at present, extremely difficult to treat. Tuberculosis symptoms are caused by infection in the lungs and require long-term antibiotic treatment. Cystic fibrosis (CF) is a condition wherein the sufferer cannot regulate the transfer of chloride ions across their membranes, particularly in the lungs. The condition invariably results in numerous, chronic, lung infections. Antibiotic treatment for either condition can lead to serious drug resistance, minimising their effectiveness. At present, antibiotics are delivered through the blood stream intra-venously, or by oral suspension/tablets, or by inhalation. Drug delivery is a big problem for CF sufferers as the antibiotic cannot efficiently transverse the lung membrane to where it is required. This leads to problems wherein resistance to the drug, through the introduction of sub-inhibitory concentrations, may become a serious issue. This makes any further treatment with the drug obsolete.

Burns patients, or patients with open wounds, are extremely susceptible to bacterial infections, notably those due to *Staphylococcal* or *Pseudomonad* species of bacteria. Treatment of such infections will invariably be by a regimen of antibiotics, either oral or intra-venously. These may be given prophylactically, or when infection is apparent. Such use of antibiotics will often lead to resistance to the drug and an ineffective treatment outcome. We envision a new method of treating burns patients using the antimicrobial compositions and methods disclosed here, using a bandage or poultice in conjunction with the described antimicrobial composition(s), that can be swapped or changed regularaly if required.

In addition, large numbers of antibiotic treatments each year are due to medical devices that have become infected whilst in use by a patient. A large number of organisms are responsible for such infections, including both Gram-positive and Gram-negative bacteria. Infections, on such items as urinary or intra-venous catheters, are often the result of the non-sterile installation of such devices. Over the course of a number of days, any bacterial cells present on the surface of the device will proliferate, leading to the production of biofilms. Such biofilms are extremely difficult to treat with antibiotics, due to the poor transfer of the drug across to the inner cells of the biofilm mass, leading often to even greater levels of tolerance of the biofilms to the antibiotic. Infection of the medical device will often require its removal and replacement, to the discomfort of the patient. Although the infection will often be noted a number of days after installation of the medical device, it will be typically incurred as the result of bacteria being present very early in the installation.

US 2007/286907 A1 discloses a method and composition for removing biofilm, disinfecting and inactivating endotoxin which generates a safe level of molecular iodine that is free of persulfate ions. The disclosed composition can be used in households, industry, on medical equipment and skin surfaces.

US 2007/292360 A1 discloses a composition for oral hygiene which is anti-plaque and treats gingivitis. The disclosed composition contains a monobasic iodide salt to provide at least 30 ppm of available iodine. The disclosed composition includes an oxidizing agent, an organic acid and a buffer. There is also disclosed a method of treating gingivitis and the removal of biofilm.

US 5639481 A discloses an enzymatically generated iodine microbicide and more specifically to the use of such a microbiocide for the inactivation of pathogenic organisms that are contaminants in or on sensitive biological materials such as the human eye or tissue culture cells.

### Object of the Invention

A first object of the disclosure is to provide a method and composition for the prevention or treatment of microbial infections. A further object is to provide a composition, which is capable of killing, as opposed to slowing the growth of, bacteria, viruses, fungi and yeasts. A still further object is to provide a composition that can kill antibiotic resistant organisms.

A further objective is to provide a composition for the treatment of both clinical and sub-clinical mastitis, without the use of antibiotics. A further object of the invention is to provide a treatment for mastitis, which does not require that the milk be discarded during treatment.

A further object of the invention is to provide a clinical solution to mastitis, which would be easy to administer.

A further object of the disclosure is to provide an antibacterial pharmaceutical composition suitable for wound care, and killing bacteria colonised in, or on, the body.

At present, there is no product available that has been shown to be effective at killing mastitis causing pathogens and curing the infection, while also allowing milk produced during treatment to be immediately processed for use in, for example, cheese and yoghurt production. The object of the invention is to produce such a treatment. A further object is that the treatment does not result in the acquisition of antibiotic resistance in the target pathogens, which provides a major benefit to veterinary and human medicine.

### Summary of the invention

The present invention provides a pharmaceutical composition for use in a method of treating or preventing mastitis in ruminants, wherein the composition comprises iodide (I-) and a source of hydrogen peroxide, together with a pharmaceutically effective carrier or diluent; wherein the pharmaceutical composition does not include a peroxidase enzyme.

According to the present invention there is provided a pharmaceutical composition comprising iodide (I-) and a source of hydrogen peroxide, together with a pharmaceutically effective carrier or diluent. The concentration of hydrogen peroxide may be less than 1% based on weight/volume or weight/weight. Preferably the pharmaceutical composition does not include a peroxidase enzyme or starch.

The pharmaceutical composition may comprise a 0.2:1 to 3:1 ratio of iodide to hydrogen peroxide by weight. The ratio may be 0.38:1 to 1.52:1 ratio iodide to hydrogen peroxide by weight. The ratio may be 0.5 : 1 to 2.5 : 1 or 0.75 : 1 to 2.0 : 1.

The ratio may be selected from:-
Between 0.2:1 and 3:1 by weight iodide to hydrogen peroxide, more preferably between 0.38:1 and 1.52:1 by weight iodide to hydrogen peroxide, or more preferably 0.76:1 by weight iodide to hydrogen peroxide.

The pharmaceutical composition may be adapted to reach a minimum concentration of 50 mg peroxide and 37 mg iodide per litre of milk, during a milking cycle within an animal.

The source of iodide may be sodium iodide, potassium iodide, lithium iodide, caesium iodide, hydrogen iodide, rhodium iodide, or other iodide releasing compounds, or a slow-releasing form of iodide, such as iodates. As used herein, the term 'iodide' does not include iodine itself or povidoneiodine. Iodine would be toxic in blood or in the mammary gland.

The source of hydrogen peroxide may be hydrogen peroxide, or peroxide releasing percarbonates, peroxide releasing citric acid or Vitamin C, or other suitable peroxide releasing compounds, or by enzymatic means, e.g. by the reduction of sugar compounds using an enzymatic pathway. Likewise, the use of other forms (and thus other form releasing) of peroxide could include sodium peroxide, lithium peroxide. Furthermore, peroxide salts could be used, including barium oxide, sodium perborate, and also hydrogen peroxide-urea adduct. Similarly, oxygen relasing pseudo peroxides could be used, including, but not limited to, superoxides, dioygenals, ozones, and ozonides. Likewise, the use of organic peroxides may also be of use, and could include, but not limited to, peroxy acids, acyl halides, aliphatic peroxides. There are other suitable oxidising compounds that may act in a suitable manner also, such as permanganate in the form of a potassium salt.

When thiocyanate is used in the composition a ratio of between 0.2-5:1 or more preferably between 0.8-1.3:1 of thiocyanate to hydrogen peroxide may be used.

The composition may be adapted for the treatment of mastitic lactating ruminant animals.

The delivery of the peroxide and iodide components may be concurrent, or sequential and/or may be by means of an intra-mammary device.

The pharmaceutically effective carrier is water, saline, an emulsion, a gel or a hydrogel.

The composition may be adapted for delivery by means of a dampened bandage.

The composition may be adapted for use as an antimicrobial nasal rinse, as an antimicrobial mouthwash, an antifungal wash, as a disinfectant, added to a bandage or poultice for the treatment of wound or burn infections, or nebulised in the form of a spray for the treatment of bacterial or fungal infection of the human or animal lung.

The disclosure also provides a medical device coated with the composition as described above, and a bandage or poultice impregnated with the composition.

Also provided is a disinfectant composition comprising iodide and a source of hydrogen peroxide.

In another aspect the invention provides a composition for use as defined herein, wherein the method of preventing or treating an infection comprises administering to a subject in need of such treatment iodide and a source of hydrogen peroxide, either concurrently or sequentially.

Here we disclose that hydrogen peroxide at low concentration (for example, a 0.003% solution), is capable of producing an effective antimicrobial therapy, by reaction with a specific concentration of iodide, ***in the absence of a peroxidase enzyme or any other synergistic agent.*** In addition to killing bacteria growing in planktonic form, such an approach may also be used to eradicate biofilms.

Compositions that contained hydrogen peroxide and iodide at concentrations of between 50-500 mg/L of each substance in water; and at weight ratios between 0.38:1 to a 1.52:1 of iodide to hydrogen peroxide by weight, and preferably in a ratio of 0.76:1 by weight of iodide to hydrogen peroxide (for example, 76 mg iodide and 100 mg hydrogen peroxide, with the 76 mg iodide provided, for example, by addition of 100 mg potassium iodide, or alternatively by addition of 90 mg sodium iodide, taking into consideration molar differences between potassium with a molecular weight of 39 and sodium with a molecular weight of 23) can be used:
a) to provide sufficient antimicrobial activity to kill both Gram-negative and Gram-positive microorganisms;
b) to eliminate bacterial biofilms growing on a variety of surfaces;
c) to avoid irritation and damage to mammalian tissue when used in a therapeutic setting;
d) to eliminate mastitis infections when infused into the mammary gland of a cow;
e) to ensure that milk produced during such treatment is free of any antimicrobial activity that could interfere with the use of milk for cheese, yoghurt or other post-processing;
f) to achieve the aforementioned targets without inducing tolerance or resistance characteristics in a target organism against the treatment itself, or against antibiotics.

The invention relates to the use of a source of antimicrobial activity (produced by a reaction between hydrogen peroxide and iodide) that can be administered once, or twice, per day for 1-15 days, preferably 1-5 days. Alternatively, an alternative source of unbound oxygen may be supplied by oxidising compounds such as permanganate.

Likewise, the invention relates to a manner of using an antimicrobial composition containing hydrogen peroxide and iodide to allow protection of the teat canal tissue in resisting colonisation by bacteria/killing bacteria on the surface of the teat canal tissue.

In one embodiment, a composition containing hydrogen peroxide, 100 mg (either directly added as hydrogen peroxide; or produced to this concentration by an alternative source of hydrogen peroxide), and potassium iodide, 100 mg, (a 0.76:1 ratio of iodide to hydrogen peroxide by weight) would be introduced into the udder environment after the animal is milked, producing antimicrobial activity, and thus killing any microbes present. Due to the reactive nature of the compounds, they must be separated (for example, in separate intra-mammary syringes or in a two-chamber syringe) until their mixture in the udder. This reaction would produce sufficient antimicrobial activity to kill the infection causing bacteria upon infusion to the empty udder. Further to this, the reaction would have ceased on milking (approximately 10-12 hours post infusion), such that post-processing of the milk could proceed normally. In another embodiment, the source of peroxide used is sodium percarbonate with a hydrogen peroxide availability of between 20 and 30%. This can be substituted easily and may provided an extended shelf life.

The invention relates to the use of two consecutive syringes to deliver the treatment.

In an alternative embodiment, the invention relates to the use of a multi-barreled syringe to deliver the treatment to the udder.

At present, teat dips are often used during milkings to help minimise the carriage of bacteria on the teat surface into the inner udder. Such an action can reduce the instance of mastitis. Iodine based compounds are often used (though these are not effective in the presence of organic material such as milk). Activated iodide-based compositions are more effective as they will work at lower concentrations. The presently discussed reaction between iodide and peroxide could be used as a preparation either as an antibacterial teat dip, or as a poultice based antibacterial barrier to an open wound (or cracked teat canal tissue).

In such a circumstance, the use of a prepared composition of hydrogen peroxide and potassium iodide gel or saline solution could be applied to a poultice or bandage. This poultice could be applied to damaged teat canal tissue or other damaged/undamaged tissues.

### Brief Description of the Drawings

Figure 1a and 1b Artificial udder model photograph and schematic. Milk was fed through the top opening by means of a peristaltic pump. Doses were infused and samples were withdrawn by means of the 'teat canal;' situated at the bottom.
Figure 2 Modified Robbins device, replete with 12 polyurethane coupons to allow attachment and proliferation of the biofilm culture. The device was provided with influent media and irrigated, through the openings at both ends.
Figure 3 Somatic Cell Count (initially, and 21 days after commencement of treatment) of 11 animals treated for two days, twice a day, with a formulation containing 100 mg potassium iodide and 100 mg hydrogen peroxide.

### Examples

1 An extemporaneous composition for use as defined herein for daily, or twice daily, treatment is made by means of intra-mammary devices; comprised of hydrogen peroxide and iodide (a range of 50-1,000 mg of hydrogen peroxide and a range of 35-700 mg of potassium iodide).
2 An extemporaneous composition for use as defined herein for daily, or twice daily, treatment is made by means of intra-mammary devices; they comprise a 0.76:1 ratio of iodide to hydrogen peroxide by weight (76 mg iodide and 100 mg of hydrogen peroxide)
3 An extemporaneous composition for use as defined herein for daily, or twice daily, treatment is made by means of intra-mammary devices; they comprise a 0.76:1 ratio of iodide to hydrogen peroxide (50 mg of hydrogen peroxide and 38 mg iodide - provided by 50 mg of potassium iodide or 45 mg sodium iodide).
4 An extemporaneous composition for use as defined herein for daily, or twice daily, treatment is made by means of intra-mammary devices; they comprise a 0.76:1 ratio of iodide to hydrogen peroxide (150 mg of hydrogen peroxide and 114 mg iodide - provided by 150 mg of potassium iodide or 135 mg sodium iodide).
5 An extemporaneous composition for use as defined herein for daily, or twice daily, treatment is made by means of intra-mammary devices; they comprise a 0.76:1 ratio of iodide to hydrogen peroxide (200 mg of hydrogen peroxide and 152 mg iodide - provided by 200 mg of potassium iodide or 180 mg sodium iodide).
6 An extemporaneous composition for use as defined herein for daily, or twice daily, treatment is made by means of intra-mammary devices; they comprise a 0.76:1 ratio of iodide to hydrogen peroxide (300 mg of hydrogen peroxide and 228 mg iodide - provided by 300 mg of potassium iodide or 270 mg sodium iodide).
7 An extemporaneous composition for use as defined herein for daily, or twice daily, treatment is made by means of intra-mammary devices; they comprise a 0.76:1 ratio of iodide to hydrogen peroxide (400 mg of hydrogen peroxide and 304 mg iodide - provided by 400 mg of potassium iodide or 360 mg sodium iodide).
8 An extemporaneous composition for use as defined herein for daily, or twice daily, treatment is made by means of intra-mammary devices; they comprise a 0.76:1 ratio of iodide to hydrogen peroxide (500 mg of hydrogen peroxide and 380 mg iodide - provided by 500 mg of potassium iodide or 450 mg sodium iodide).
9 An extemporaneous composition for use as defined herein for daily, or twice daily, treatment is made by means of intra-mammary devices; they comprise a 0.76:1 ratio of iodide to hydrogen peroxide (1,000 mg of hydrogen peroxide and 760 mg iodide - provided by 1,000 mg of potassium iodide or 900 mg of sodium iodide).
10 The above compositions (1-9) where the peroxide is present as a peroxide releasing compound such as percarbonates or perhydrates. In such embodiments, the appropriate concentration of percarbonate can be calculated by the available peroxide content (typically 20-30%) of the percarbonate and the required concentration of peroxide. For example, 1000 mg of sodium percarbonate with 30% available hydrogen peroxide would release 300 mg hydrogen peroxide on dissolving in the environment. Similarly the use of 333 mg percarbonate would release 100 mg hydrogen peroxide.
11 A composition for use as defined herein used to produce a general antibacterial wash, used for example as a teat dip wash, containing freshly mixed source of hydrogen peroxide and iodide at a range of 10-10,000 mg hydrogen peroxide and 7.6-7,600 mg iodide.
12 A composition for use as defined herein used a general antibacterial wash, for example as a teat dip wash, containing freshly mixed source of ionic iodide wherein 19-7,600 mg iodide per litre is present (provided by 25-10,000 mg potassium iodide or 22.5-9,000 mg sodium iodide), and prepared at a ratio of between 0.38-1.52:1 of iodide to hydrogen peroxide.
13 A composition for use as defined herein used a general antibacterial wash, for example as a teat dip wash, containing freshly mixed source of hydrogen peroxide and iodide wherein 100 mg hydrogen peroxide per litre is present and 76 mg iodide per litre is present (provided by 100 mg potassium iodide or 90 mg sodium iodide).
14 A composition for use as defined herein used a general antibacterial wash, for example as a teat dip wash, containing freshly mixed source of hydrogen peroxide and iodide wherein 200 mg hydrogen peroxide per litre is present and 152 mg iodide per litre is present - provided by 200 mg potassium iodide or 180 mg sodium iodide.
15 The disclosure provides a poultice prepared for wound care where the poultice is dipped into the antibacterial wash described in examples above (12-14).
16 A compoisition as described above where the source of hydrogen peroxide is additionally, or alternatively, be provided by a peroxide releasing compound, such as percarbonate, or by enzymatic means, e.g. by the reduction of sugar compounds, sufficient to produce the required concentration of hydrogen peroxide. Furthermore, compositions that include an alternative appropriate oxidising compound to start the reaction, such as potassium permanganate, are included.

The source of iodide could additionally, or alternatively, be provided by a list of compounds set out elsewhere in this text, including, but not limited to sodium iodide, potassium iodide, lithium iodide, caesium iodide, hydrogen iodide, rhodium iodide, or other iodide releasing compounds, or a slow-releasing form of iodide, such as a degraded iodate.

Furthermore, iodide could be replaced by a thiocyanate molecule (supplied in the form of but not being limited to, for example, sodium or potassium thiocyanate) whilst adhereing to a ratio of 0.2-5:1 or a more preferably 0.8-1.3:1 of thiocyanate to hydrogen peroxide) in areas where iodide use may pose an issue, for example, in people with thyroid iproblems.

This preparation can be administered by the use of consecutive suitable intra-mammary syringes, or a dual-barreled syringe (preventing premature reaction of iodide and peroxide).

All described compositions are to be prepared in a pharmaceutically suitable carrier. Such carriers include, but are not limited to, water, a pH suitable saline, a pH suitable saline including bicarbonate buffer, gel, or hydrogel.

Glucocorticoids from a list including, but not limited to prednisone, prednisilone, cortisol and hydrocortisone could be supplemented to the composition to aid with local inflammation issues during infection. For example, supplementation of 10-20 mg prednisolone or prednisone or 30-60 mg hydrocortisone to the antimicrobial composition could be used to counteract infection inflammation as part of a mastitis treatment.

The application of the composition will dictate the use of carrier. For example, mastitis intra-mammary treatment could be prepared in a saline or water, or in a more complex solution such as stearate/oil. A stearate/oil-based composition would be suitable in compositions to include the hydrogen peroxide-releasing sodium percarbonate as it prevents release of peroxide until the composition is diluted in milk in the mammmary gland.

Furthermore, a hydrogel-based composition could be used to aid application in a wound care/bandage setting. Such an example is a sodium polyacryalte based hydrogel with a pH adjusted composition. Moreover, a saline or bicarbonate/saline carrier could be used in a disinfectant setting or for use as a nasal rinse to relieve sinusitis and accompanying infections.

In addition, the use of a temperature sensitive hydrogel containing poultice could be used to sequester the components such that application of the poultice to tissue at body temperature would allow the release of antimicrobial activity as the components react.

### Pharmacological Results

### Result 1

A freshly prepared mixture of hydrogen peroxide and potassium iodide was mixed (100 mg of each, at a ratio of iodide to hydrogen peroxide of 0.76:1 by weight) in 10 ml sterile water. Using a micro-broth dilution technique, the composition was doubly diluted in broth containing the test bacterial organism using a 96 well plate system. Depending on the sensitivity of the organism, a cut-off point was reached at a dilution at which the bacterial culture was no longer able to resist the effects of the antimicrobial composition. This is characterised as the minimum inhibitory concentration (MIC). The lower the MIC, the more sensitive the organism.

**Table 1. MIC of mastitis causing organisms to an antimicrobial composition containing 100 mg hydrogen peroxide and 100 mg potassium iodide prepared freshly in a 10 ml volume and diluted accordingly. P. aeruginosa PA-29 exhibited increased tolerance to biocides and resistance to fluoroquinolone classed antibiotics. P. aeruginosa 'R' strains are cystic fibrosis clinical isolates that exhibited tolerances one or more of amakcin, tobramycin, ciprofloxacin, and gentamicin. S. aureus BH1CC was described elsewhere as an MRSA class organism.**

| Strain | MIC (mg/L of available iodide/peroxide |
|---|---|
| *Escherichia coli* ATCC 25922 | 20-40 |
| *Streptococcus dysgoloctiae* 143 (mastitis isolate) | 5-10 |
| *Streptococcus dysgalactiae* 160 (mastitis isolate) | 5-10 |
| *Streptococcus uberis* (mastitis isolate) | 5-10 |
| *Staphylococcus aureus* 15676 (mastitis isolate) | 20-40 |
| *Staphylococcus aureus* BH1CC (Rudkin *et al.,* 2012) | 20-40 |
| Non-haemolytic coliform (mastitis isolate) | 20-40 |
| *P. aeruginosa* PA01 (wild type) | 25-50 |
| *P. aeruginosa* R550/2012 9026 (resistant CF isolate) | 25-50 |
| *P. aeruginosa* R468/2012 9027 (resistant CF isolate) | 25-50 |
| *P. aeruginosa* R479/2012 9028 (resistant CF isolate) | 25-50 |
| *P. aeruginosa* R480/2012 9029 (resistant CF isolate) | 25-50 |
| *P. aeruginosa PA-29* (McCay *et al.,* 2010) | 25-50 |
| *Candida albicans* | 20-60 |
| *Candida tropicalis* | 20-60 |
| *Candida glabrata* | 20-60 |
| *Candida krusei* | 20-60 |
| *Saccharomyces cerevisiae* | 20-60 |

Table 1 shows the sensitivity of a number of mastitis-causing organisms to antimicrobial activity produced as a result of the reaction between hydrogen peroxide and iodide in the absence of a peroxidase enzyme. As is evident, all strains tested, including the Gram-positive organisms, were sensitive to the composition. It is evident that, in broth, the use of lactoperoxidase is not required. In addition, it is evident that the composition is also effective against fungi.

Furthermore, the broths were sub-cultured at various times post testing to assay for cell viability. This was carried out by sub-culturing 100 microlitres of the treated culture to 10 ml of fresh broth containing no antimicrobial. This differentiated between cell death and cell stasis (where viable cells would be detected). No growth was observed after 48, 72 or 96 hours post treatment (using concentrations in the same order of magnitude as those inhibiting them in the MIC test). This demonstrates that the produced antimicrobial activity is bacteriocidal to both Gram-negative and Gram-positive organisms, a surprising finding.

### Result 2

The activity of the antimicrobial composition of the invention (peroxide and iodide) and another composition containing peroxide and thiocyanate against bacterial cells growing in biofilm mode was established by means of a modified Robbin's device (MRD). A biofilm of *E. coli, S. aureus, P. aeruginosa,* or a mixture of all three was established by inoculating 500 ml LB in a 1 L glass bottle that was connected to a modified Robbins device. The MRD system consists of 12 sampling ports, to which medical-grade polyurethane coupons (50 mm² surface area) were inserted. The culture was allowed to proliferate at 37°C and the MRD was fed/irrigated with culture at a rate of 0.1 h⁻¹ for at least 48 hours (a further 100 ml LB were supplemented after the initial 24 hours). The MRD was then irrigated with a control or a test solution. A small amount of constant force was used in the irrigation, such that it took approximately **90 seconds** for the complete volume to pass through the system. The control used was a physiological saline solution. A number of distinct solutions were tested by irrigating the MRD chamber whilst the coupons were *in situ*: a '1x' solution containing 0.3 g I⁻¹ potassium thiocyanate and/or potassium iodide, and 0.003% hydrogen peroxide; a '5x' solution containing 1.5 g I⁻¹ potassium thiocyanate and/or potassium iodide, and 0.015% hydrogen peroxide; and a '20x' solution containing 6 g I⁻¹ potassium thiocyanate and/or potassium iodide, and 0.06% hydrogen peroxide.

**Table 2 Total viable counts (colony forming units per coupon) isolated from irrigated/non-irrigated E. coli biofilm and tested against a thiocyanate-based solution. Values represent the range from four coupons.**

| *Escherichia coli* | 1x | 5x | 20x |
|---|---|---|---|
| No irrigation | 10⁵⁻⁶ | 10⁵⁻⁶ | 10⁵⁻⁶ |
| Saline | 10⁵⁻⁶ | 10⁵⁻⁶ | 10⁵⁻⁶ |
| Saline + SCN/H₂O₂ | 10⁵⁻⁶ | 10⁵⁻⁶ | 10⁴⁻⁵ |

From Table 2 it is clear that 10⁵⁻⁶ colony forming units (CFU) were recoverable from the coupons in the absence of an irrigation step. Likewise, the bacterial viability was not significantly altered when the system was irrigated with a saline solution. The supplementation of thiocyanate and hydrogen peroxide to the solution at either 1x or 5x levels did not alter the outcome in cell viability. The use of a 20x solution did begin to affect cell viability, with a log-reduction in numbers typically noted. Similar results were noted when repeated with *P. aeruginosa* and *S. aureus* biofilm cultures).

**Table 3 Total viable counts (colony forming units per coupon) isolated from irrigated/non-irrigated E. coli biofilm using an iodide-based solution. Values represent the mean from four replicates and two biological replicates. NG - no viable cells recoverable**

| *Escherichia coli* | 1x | 5x | 20x |
|---|---|---|---|
| No irrigation | 8.3(±0.8)x10⁵ | 2.2(±0.4)x10⁶ | 2.3(±0.8)x10⁶ |
| Saline | 8.2(±0.9)x10⁵ | 3.8(±0.2)x10⁶ | 1.9(±0.5)x10⁶ |
| Saline + Iodide/H₂O₂ | 5(±1)x10⁴ | 3.3(±0.5)x10³ | NG |

The use of iodide instead of peroxide and thiocyanate derived OSCN⁻ had a profound effect on the outcome of the test (Table 3), against all three organisms. Cell viability was notably lower on use of a 1x iodide-based solution. Typical 100-1000-fold reductions in cell viability were noted after the 90 seconds irrigation (Table 3). A reduction from 10⁶ CFU to 10³ CFU was observed for *E. coli* on using the iodide at a '5x' level. It should be noted that three of the four samples yielded no recoverable cells at all. A '20x' solution was sufficient to result in a complete lack of viable cell recovery (Table 3). Repetition of the set-up, but using a mixed culture of *E. coli* ATCC 25922, *S. aureus* DSM 15676 and *P. aeruginosa* NCIMB 10421 resulted in a very similar pattern (Table 4). A '1x' solution was sufficient to result in a 100-fold reduction in bacterial viability. Both the '5x' and '20x' solutions were sufficient to result in a complete lack of viable cells recoverable from the coupon surface. Attachment to the surface was greater on using a mixed culture and there was some evidence to suggest that some cells were lost on irrigating with the saline solution only. Such a decrease in viability of biofilm attached cells within such a short space of time (90 seconds) would teach that the iodide/hydrogen peroxide composition would be very effective as a therapeutic in biofilm centred infections when involving an *in vivo* model.

**Table 4 Total viable counts (colony forming units per coupon) isolated from irrigated/non-irrigated mixed biofilm of E. coli, P. aeruginosa, and S. aureus cultured together and tested against an activate-iodide solution. Values represent the mean from four replicates and two biological replicates. NG - no viable cells recoverable**

| *E.coli, Staph, P.aerug* | 1x | 5x | 20x |
|---|---|---|---|
| No irrigation | 8.6 (±1)x10⁶ | 7(±1.3)x10⁶ | 2(±0.4) x10⁷ |
| Saline | 2.6(±0.6)x10⁷ | 1.9(±0.4)x10⁶ | 5.9(±1.3) x 0⁶ |
| Saline + Iodide/H₂O₂ | 5.9(±1.1)x10⁴ | NG | NG |

The data presented in Tables 3 and 4 would strongly indicate that the activity produced by even very low concentrations of the antimicrobial composition was capable of killing biofilm-formed bacterial cultures of both Gram-negative and Gram-positive organisms and even mixed culture consortia. This result would compare favourably to the use of antibiotics to achieve the same outcome. Many of the antibiotics would require 1,000-fold increases in concentration to achieve kills of biofilm cells by comparison to planktonic cells (i.e. free-floating). This was not the case when an iodide/hydrogen peroxide composition was employed to produce the antimicrobial activity.

We disclose that that hydrogen peroxide, even at low concentrations (e.g. a 0.003% solution), is capable of producing an effective antimicrobial action against infectious disease causing organisms, by reaction with iodide in the absence of a peroxidase enzyme. An increase in contact time (for example, 10 minutes) would allow 1x solutions to kill the bacterial cultures to the same degree noted here for stronger concentrations.

The prior art teaches that even higher concentrations of an antimicrobial agent will be required to effectively kill biofilm bacteria. The amount of hydrogen peroxide in the present invention is effective, however, when used with the correct amount of iodide. The composition of the invention will provide (a) a greater kill efficiency; (b) effective biofilm eradication and (c) a composition suitable for in vivo applications, such as treatment of mastitis or other infections (and doing so without causing irritation to the infection site).

### Result 3

Lactoperoxidase has been shown to be present at significant concentrations in bovine milk, up to 30 mg I⁻¹. This level will depend on the health of the animal, breed of the cow etc. and therefore cannot be depended on to be present at optimal levels for an individual animal treatment. A reliance on naturally present lactoperoxidase would not allow an optimally designed treatment. To demonstrate that the described method and composition disclosed herein does not require endogenous lactoperoxidase, MICs were carried out in a broth, raw milk, pasteurised milk, and ultra heat treated (UHT) milk. The lactoperoxidase enzyme is inactivated at elevated temperatures, such as those used in the pasteurisation process. Typical pasteurisation processes (72°C for 15 seconds) reduce activity of the enzyme by a significant amount (c. 70%), while treatment at 80°C or more (including UHT treatment at 135°C) would result in complete destruction of the enzyme. Were lactoperoxidase present in the milk to be essential to the reaction, the use of low concentrations of iodide and peroxide in a UHT milk environment should not produce any noted antimicrobial effect.

On testing the composition of the invention in these environments, *E. coli* was no more tolerant to the effects in UHT milk than that in broth, raw milk, and pasteurised milk (See Table 5). This clearly indicates that the composition of the invention is not in any way reliant on the presence of endogenous or native lactoperoxidase enzyme.

Furthermore, the supplementation of lactoperoxidase to the broths of UHT milk concentrations did not affect the outcome of the minimum inhibitory concentration determinations after 24 hours incubation (Table 5).

**Table 5 MICs of E. coli ATCC 25922 to appropriately diluted formulation of 100 mg hydrogen peroxide and 100 mg potassium iodide. Values represent minimum mg/L value of potassium iodide and hydrogen peroxide required to inhibit growth over 24 hours. LB - lysogeny broth. Pasteurised milk - 72° C for 15 seconds. UHT milk - 135° C for 3 seconds. LP - lactoperoxidase.**

| Test medium | MIC (mg/L of available iodide/peroxide |
|---|---|
| Water | 20-40 |
| LB broth | 25-50 |
| LB broth + 100 Units activity/L LP | 25-50 |
| LB broth + 500 Units activity/L LP | 25-50 |
| Raw Milk | 25-50 |
| Pasteurised Milk | 25-50 |
| UHT Milk | 25-50 |
| UHT Milk + 200 Units activity/L LP | 25-50 |

### Result 4

In an attempt to induce a tolerance to an antimicrobial, the micro-broth dilution method was utilised, with repeated passaging, with *E. coli* ATCC 25922 and a number of antibiotics, as well as the composition of the invention containing hydrogen peroxide and potassium iodide. Stock concentrations of kanamycin, polymyxin B, and levofloxacin (all clinically important and relevant antibiotics) were used to demonstrate the development of typical resistance characteristics to antibiotics. Doubling dilutions of antimicrobial compositions were made using a 96 well plate. The well contents were inoculated with 10⁴ cfu ml⁻¹ *E. coli* ATCC 25922 and allowed to incubate. The well with the highest concentration of antimicrobial that still resulted in growth overnight served as the inoculum for the next day's passage. Therefore, for example, the 'kanamycin-passaged' strain did not come into contact with any of the antimicrobials during the test other than kanamycin.

After only 8 passages in the presence of the antibiotics, the organism was completely resistant to the kanamycin and polymyxin B drugs. After 10 passages, the organism was 8 times more tolerant to the effects of levofloxacin than it was pre-passaging. These results mirror the use of antibiotics in the environment wherein a bacterium will gain resistance due to repeated exposure to the antibiotics. However, during the same experiment, the organism was no more resistant to the effects of the antimicrobial activity produced by the hydrogen peroxide/iodide composition, after a similar number of passages. This is due to the inherent mode of action wherein there is no single target for the antimicrobial activity produced by this reaction, acting as is does across a variety of bacterial proteins. A bacterial cell would require multiple, concurrent, mutations to gain resistance, whilst only requiring one mutation in response to traditional antibiotics. This severely limits the ability of the organism to counter-act the inventions actions. Broad range acting antimicrobials have been described as unlikely to illicit bacterial resistance, or for development of cross-resistance toward antibiotics. The antimicrobial composition was also capable of killing a number of antibiotic-resistant *P. aeruginosa* bacteria. The developed kanamycin and polymyxin B resistant strains, as well as the levofloxain tolerant mutant, were no more tolerant to the actions of the disclosed composition, indicating the cross-resistance would not occur. A strain described previously by Mc Cay et al., (Microbiology, Vol. 156, No.1 30-38, 2010) as being resistant to fluoroquinolone and tolerant to benzalkonium chloride biocide was sensitive to the actions of the antimicrobial activity of the invention (Table 1). Likewise, a number of clinical cystic fibrosis isolated *P. aeruginosa* strains, with elevated tolerances to key antibiotics used in the treatment of CF patients, were no more tolerant to the antimicrobial activity than the *P. aeruginosa* wild type strain (see Table 1). Further to this, S. *aureus* BH1CC (MRSA) is an antibiotic resistant strain (oxacillin MIC > 256 mg/L) isolated from Beaumont hospital, Dublin, Ireland. The strain was no more tolerant to the actions of the reaction than another *Staph. aureus* strain (Table 1). This would again indicate that the method and composition disclosed here would be effective against MRSA isolates.

### Result 5

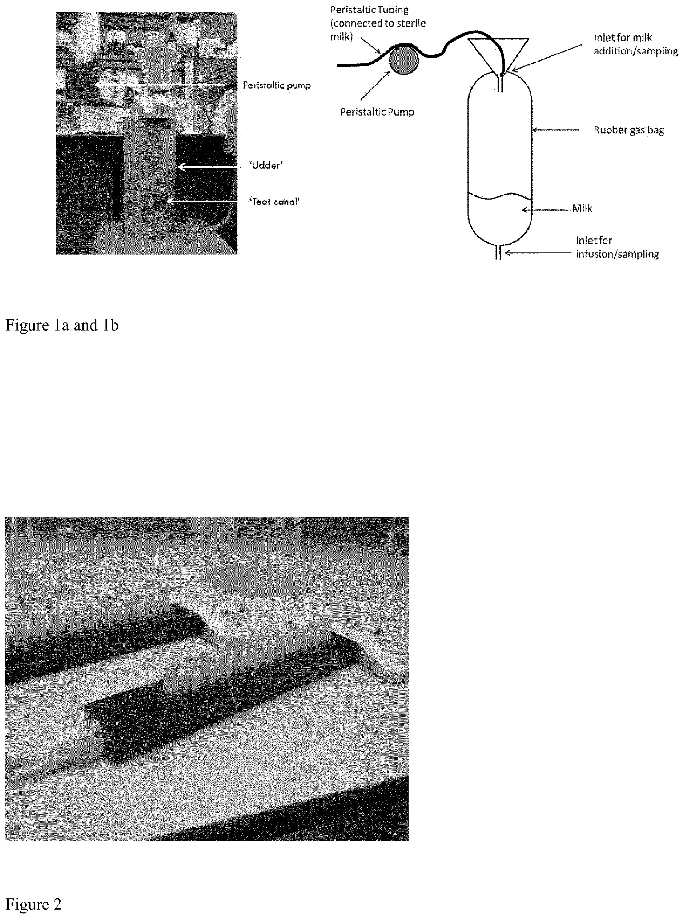

An experimental method was devised to determine the most appropriate concentration of the composition required to provide antimicrobial action within the udder, but not persisting in the milk, post milking of the animal. A simulated bovine udder was designed, using an amended rubber gas-collection bag with two inlet channels (see Figure 1). Various formulations, produced freshly by means of hydrogen peroxide and potassium iodide reactions, were introduced into the bag through an inlet. To simulate the udder environment, milk was then fed into the udder at the same rate a typical cow might produce milk, 12 L a day. Samples of the milk were taken routinely at various time points and the antimicrobial activity of the milk was determined by the spiking of the sample with *E*. *coli.* The spiked milk sample was then incubated and subsequently tested for the presence of remaining viable *E. coli* cells. A lack of viable cells indicated that there was sufficient antimicrobial activity present to inhibit/kill the *E. coli.* Conversely, the presence of viable cells indicated that the milk no longer contained sufficient quantities of antimicrobial activity to inhibit the growth of the bacterium. Using this method, each prospective embodiment was titred, such that activity was present for at least 5-6 hours post treatment/infusion, but no longer than 10 hours following treatment (such that activity will be absent when the milk is outside the udder environment). A 10 ml volume containing 100 mg potassium iodide, and 100 mg hydrogen peroxide achieved the desired characteristic (a 0.76:1 ratio of iodide to hydrogen peroxide by weight). At peroxide concentrations less than 50 mg (i.e. a >1.52:1 ratio of iodide to peroxide by weight), there was insufficient antimicrobial activity over a 4-5 hour period to be an effective treatment. At peroxide concentrations greater than 300 mg (a <0.38:1 ratio of iodide to peroxide by weight), antimicrobial activity persisted for greater than 10 hours, which could be a potential problem for post-processing of milk. The risk of damage to animal cell tissue also increases with increased peroxide concentrations. A ratio of between 0.38-1.52:1 of iodide to hydrogen peroxide by weight was suitable in providing a suitably paced reaction in the simulated cow udder.

Using this method, a suitable ratio of peroxide to iodide was predicted for the treatment various ruminant animals (cows, sheep, etc.). Interestingly, an undiluted 10 ml composition of peroxide and iodide (200 mg of each at a ratio of 0.76:1 iodide:hydrogen peroxide by weight) was found to have lost all significant antimicrobial activity within a smaller time frame than useful in a mastitis setting (though still useful for disinfectant purposes where contact time is much shorter), despite the elevated initial concentrations. This finding confirms that consideration of the kinetics of the reactions between hydrogen peroxide and iodide was a prerequisite for the development of suitable compositions for therapeutic purposes. It is counter-intuitive that a more concentrated solution of an antimicrobial compound would be less effective than a more dilute version and that the more concentrated solution would lose activity after a shorter period of time, by comparison to the embodiment containing 100 mg of each substance - which again emphasises the link between reaction kinetics and efficacy in this case.

Failure to take into account the dynamic volume changes in the udder environment is also a problem with respect to producing an effective mastitis therapy. For example, a composition (100 mg potassium iodide and 50 mg hydrogen peroxide - a 1.52:1 ratio by weight of iodide to hydrogen peroxide) was added to a constant, unchanging volume of milk of 1 L. The composition remained antimicrobially active for >24 hours in this situation, but the same composition recorded antimicrobial activity for <3 hours during tests in the udder model. Such a result, in the absence of an appropriate simulated udder model method, would teach that the antimicrobial composition was active for too long (i.e. greater than 10 hours) using this particular ratio and that activity would be expected long after the milk was taken from the udder. This proved that a simulated udder model, as disclosed in this application, provided an effective method, which allowed the design of an appropriate therapeutic for *in vivo* applications.

### Result 6

The correct concentrations of peroxide and iodide are required to ensure an effective therapy for mastitis and to ensure that the post-processing of milk is not affected. The use of the model udder system allowed us to develop the correct concentration of composition. If an incorrect concentration is used the same results will not be observed.

For example, a lactating dairy cow was infused with a composition containing both potassium iodide and hydrogen peroxide (5 ml solution of each, containing 50 mg (converting to 38 mg iodide) and 150 mg, respectively - a ratio of 0.25:1 of iodide to peroxide by weight). A 5 ml embodiment was infused into lactating cow's udders, twice a day for 2 days. The animals were monitored for changes in activity, milk yield etc. Irritation, reduced milk yield and udder sensitivity was noted in the animals, indicating that the production of the antimicrobial activity was insufficient (or too slow) and that un-utilised peroxide was remaining, which started to irritate or damage the animal tissue. This experiment used a concentration of 0.1-0.15% peroxide in the udder, which although significantly lower than the concentrations of peroxide reported to be required in antiseptics, still resulted in irritation in the animals. A treatment for mastitis by use of such an embodiment would be unacceptable. Use of this embodiment (or a similar one where the peroxide is present for a prolonged period) would also lead to issues in post-processing of milk as well as initial irritation in the animal. This is because the presence of peroxide would likely inhibit any starter cultures used in the cheese and yogurt processing stages.

To demonstrate that the use of an alternative and preferred embodiment (containing 100 mg potassium iodide and 100 mg hydrogen peroxide - a 0.76:1 ratio of iodide to peroxide by weight) would not harm animals, 5 lactating dairy cows were infused with this embodiment, twice a day, for 5 days. The animals were monitored for changes in activity, milk yield, milk quality, etc during the period immediately following treatment, and for several months after treatment. No signs of discomfort or changes in udder sensitivity, hardness or temperature were noted following treatment; and there was no change in the quality (nutritional, chemical or biological) or quantity of the milk produced by the individual animals. This demonstrated that: a) the composition and treatment was not toxic to the animal at the level described (even after a prolonged, 5 day treatment); b) there was no 'bottle neck' in the reaction to produce antimicrobial activity resulting in a prolonged presence of peroxide; and c) that the likely cause of the irritation described earlier was the prolonged exposure of the teat canal tissue to excessive levels of peroxide. These specific results also support the suitability of this embodiment of the therapy for use as a treatment for sub-clinical mastitis and/or as a prophylactic therapy, given the absence of any negative effects on the cow and the possibility for continued utilisation of milk produced by an animal undergoing treatment.

### Result 7

To demonstrate that the treatment method would not interfere with post-processing, milk taken from healthy animals, infused with a composition containing 100 mg potassium iodide and 100 mg hydrogen peroxide, was tested using a Delvo test. This is the 'gold standard' for testing for the presence of antimicrobials (sensitive to parts per billion level for some antimicrobials and to a broad range of compounds). Milk produced from healthy cows and from cows with clinical mastitis, which were treated with the composition, recorded a 'negative' result for antimicrobials when tested during treatment, thus proving that the antimicrobial composition was active for a limited period of time, i.e. between milkings only. Critically, this demonstrates the embodiment would not affect post-processing of the milk - if there is insufficient antimicrobial activity present immediately post-milking to inhibit the Delvo test organism, then there is no issue with respect to the inhibition of starter cultures.

### Result 8

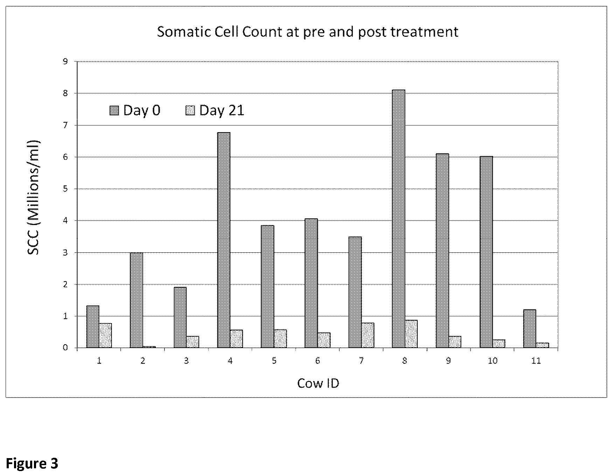

A number of sick animals were identified that were suffering from chronic clinical mastitis due to S. *aureus* infections. These animals were non-responsive to previous antibiotic therapies in the months prior to the trial and demonstrated the typical cyclical SCC increases associated with chronic mastitis. Eleven of these animals were treated with an embodiment of the present invention, containing 100 mg potassium iodide and 100 mg hydrogen peroxide (0.76:1 by weight). The animals were treated twice a day, post milking, for two days. The somatic cell count was determined on day 21 post treatment start. This is in line with European Medicines Agency guideline dates (ensuring any effect noted is not merely transitory). At day 21, there was no evidence of any remaining clinical infection based on milk SCC counts, udder sensitivity, swelling, temperature or other milk quality indicators, thus demonstrating the therapeutic effectiveness of the method, even in cows chronically infected with *S. aureus* (see Figure 3 for SCC count data). This trial demonstrates that the composition was capable of treating *S. aureus* infections of a chronic nature. Crucially, this also demonstrates that the composition was not harmful to the animals, as may be the case on using hydrogen peroxide. These animals were due to be culled (low milk yield, non response to numerous antibiotics, and continuous elevated SCC) but were returned to the herd and were milking normally for months following treatment using the invention. The estimated saving to the farmer due to increased milk production, no milk discard, and veterinarian fees, and replacement of animals was >€10,000 ($13,000).

A novel finding from this *in vivo* study and the *in vitro* studies reported in this application using milk as bacterial growth medium - is the ability of the hydrogen peroxide/iodide model to function *in vivo* or *in vitro* in milk containing relatively elevated levels of thiocyanate. This would not be the case were a peroxidase enzyme employed to catalyse the reaction betwen hydrogen peroxide and iodide, based on the prior art.

Klebanoff et al., (J Exp Med 1967 126(6):1063-78) describe the fact that "*thiocyanate ions were inhibitory*" to the actions of IO⁻, produced by the peroxidase-catalysed reaction between hydrogen peroxide and iodide. The authors of that study believed this was an "*apparent paradox*" given the role of thiocyanate in the production of OSCN⁻. Such a finding would further teach that the efficacy of the peroxidase-catalysed reaction betwen hydrogen peroxide and iodide to produce IO⁻ as an antimicrobial in the udder environment would be limited, as thiocyanate is present at significant concentrations in bovine milk (WHO/FAO, 2005). Tenovuo et *al.* and the articles discussed in that review (Oral Diseases {2002} 8, 23-29) stated that although an oxidation products of iodide are much more potent than thiocyanate oxidation products, the "*major problem with iodide in connection with LP-system is that even relatively small concentrations of salivary thiocyanate abolish the bacteriocidal effect of the LP*/*I-*/*H₂O₂ systems as SCN- is the preferred substrate*". Moreover, it has been reported that a similar and strong interference between thiocyanate and iodide in an oral biofilm study of the yeast, *Candida albicans* strongly and negatively affects IO⁻ production. The authors state that *"the ability of IO⁻ to affect yeast growth and survival must be questioned in regard of the other iodine compounds, since clinical trials have shown limited or weak beneficial effects of other iodine formulations used for in vivo antifungal purposes".* And *"The efficiency of an iodide*/*peroxidase system demonstrated* in vitro *through this investigation is difficult to transfer to either animal or human. Beside the toxicity of oxidant products on host cells and the immunogenicity of enzymes isolated for example from bovine milk, the oxidation of iodide is under the control of thiocyanate, which is not only present in several exocrine secretions (for example in human saliva) but is also preferentially used as substrate by lactoperoxidase. Indeed, simultaneous incorporation of both substrates in the same gel provided a decrease of the beneficial effect of 2 mM iodide in the presence of increasing concentrations of thiocyanate ranging from 0.25 to 4 mM, which correspond to the normal range of thiocyanate in saliva"* (Ahariz and Courtois 2010. Clinical, Cosmetic and Investigational Dentistry 2010:2 69-78). These authors conclude that peroxidase-generated OI⁻ to prevent *C*. *albicans* biofilm development would only be useful for treatment of devices in *ex vivo* conditions.

The concentrations of thiocyanate in milk are high, relative to the teaching described above, and they vary widely according to the animal diet. A severe inhibition of IO⁻ production by thiocyanate would thus suggest that a therapy based on iodide and hydrogen peroxide would be of no benefit for the treatment of mastitis.

The results from the described clinical trials of Result 8 carried out by the applicants recorded no negative impact of innate thiocyanate in milk on the efficacy of the proposed therapy, a highly surprising finding given the previous cited literature. It is explained by the need to balance the concentrations of the reactants appropriately, using the method described in the present invention, and of the critical importance of reaction kinetics to the antimicrobial efficacy of a therapy based on the antimicrobial activity produced by the reaction between peroxide and iodide.

### Result 9

The simulated udder model method represents a significant advance on the prior art with respect to the assessment of antimicrobial activity. Results 6 and 8 above describe the use of the therapy with 100 mg potassium iodide and 100 mg hydrogen peroxide (0.76 iodide:1 hydrogen peroxide by weight). Use of too low a concentration of components resulted in no irritation or other negative effects to the animal, but also no significant drop in SCC counts or improvement in infection, during or post treatment. *In vitro* experiments using **traditional** minimum inhibitory concentration testing had, however, suggested that the solution would be antimicrobially active for c. 24 hours in a 1 L volume. The simulated udder model method correctly predicted, however, that it would not be antimicrobial for a sufficient period of time *in vivo* to result in a sufficient kill of the pathogen. The *in vivo* data validated the predictions of the experimental model and demonstrated its importance in mapping the kinetics of the udder environment. It is now evident that there is a range of concentrations and ratios that must be achieved in the reaction to be effective for use as a mastitis treatment, and that the described embodiment in Result 8 achieves this effective concentration for the treatment of bovine mastitis.

It is evident upon examining these results, that the described hydrogen peroxide/iodide composition is effective at killing a wide range of organisms (Table 1). It has been shown also that there was a significant difference in outcome between using this model, by comparison to one based on the production of OSCN⁻ with respect to treatment of biofilm cultures of both Gram-positive and Gram-negative organisms (Tables 2-4). The ratio of the compounds has also been shown to be extremely important at an *in vivo* level. Too high a concentration leads to tissue damage or prolonged activity (affecting post-processing of milk; failure of Delvo test). Too low a concentration or ratio leads to insufficient activity, minimising the effect of the treatment in killing the present pathogens. The kinetics of the udder environment, starting from a very low volume and increasing to ^{∼}1-4 litres of milk is also an important factor. Surprisingly, undiluted compositions lost activity much quicker than diluted compositions. Traditional *in vitro* models using a constant final volume would teach one to over-estimate the effectiveness of the hydrogen peroxide/iodide model and would not translate to success *in vivo* - this is a feature of the prior art cited in this application. The development, design and deployment of the novel simulated udder model method disclosed here allowed the proper evaluation of the disclosed treatment and ultimately enabled the development of the novel therapy for mastitis.

This is the first description of the reaction between low concentrations of hydrogen peroxide and iodide as being the basis for a highly efficient bacteriocidal composition against both Gram-negative and Gram-positive bacteria, independent of lactoperoxidase (or any other peroxidase enzyme) activity. It is the first to demonstrate that the even low concentrations of the reaction products of hydrogen peroxide and iodide provide an efficient means of eradicating bacterial biofilms, even in the presence of thiocyanate. It is also the first disclosure of the successful use of a hydrogen peroxide and iodide composition for therapeutic use for treatment of bovine mastitis, and in a potential open wound setting by way of a poultice.

This is the first description of an experimental method to characterise the evolving udder environment in order to allow the development of a mastitis therapy based on hydrogen peroxide and iodide. We present *in vivo* data to validate the efficacy of the novel method to produce a safe treatment for bovine mastitis, a method capable of both treating and curing mastitis, while simultaneously allowing producing of milk that will not affect the post-processing processes, such as cheese or yoghurt production.

### Result 10

To demonstrate the difference between traditional iodine based antimicrobials and the activated IO⁻ form described herein, MICs were performed in a variety of media, including water, LB broth, and milk, using *E. coli* ATCC 25922 as a test organism. As a comparison, PVP-I or povidone iodine, used frequently in hospital setting as a topical antiseptic, was used as a source of free iodine (I₂). Although the PVP-I is strongly antimicrobial in water and saline, it decomposes extremely quickly in the presence of organic material present in LB and milk (see Table 6). This limits the efficacy of PVP-I to such areas where organic material is not present. This is not the case with the present OI⁻ formations. Organic material does not irreversibly render the solution ineffective. Curiously, the use of a combined hydrogen peroxide and PVP-I in a saline environment resulted in no antimicrobial activity either, as the PVP-I apparently exerts a negative effect on peroxide antimicrobial activity (Table 6). Repetition in a broth environment causes the PVP-I to be broken down before it can affect the peroxide, and the MIC is identical to that of peroxide in the absence of PVP-I. Using the composition of 100 mg H₂O₂ and 100 mg Kl, MICs of between 20-40 mg L-1 were achieved in each of the test environments (milk, broth and water), indicating that the presence of organic material did not inhibit the desired antimicrobial activity

**Table 6 Activity of and PVP-I in absence and presence of organic material**

| **Solution environment** | **MIC (mg L⁻¹)** |
|---|---|
| PVP-I (saline) | 1-2 |
| PVP-I (in LB) | >250 |
| PVP-I (milk) | >250 |
| PVP-I + H₂O₂ (saline) | >250 |
| PVP-I + H₂O₂ (broth) | 20-40 |
| H₂O₂ (broth) | 20-40 |

## Claims

1. A pharmaceutical composition for use in a method of treating or preventing mastitis in ruminants, wherein the composition comprises iodide (I-) and a source of hydrogen peroxide, together with a pharmaceutically effective carrier or diluent; wherein the pharmaceutical composition does not include a peroxidase enzyme.

2. A pharmaceutical composition for use according to claim 1, where the concentration of hydrogen peroxide is less than 1% based on weight/volume or weight/weight.

3. A pharmaceutical composition for use according to any preceding claim comprising a 0.2 : 1 to 3 : 1 ratio by weight of iodide to hydrogen peroxide.

4. A pharmaceutical composition for use according to any preceding claim comprising a 0.38 : 1 to 1.52 : 1 ratio by weight of iodide to hydrogen peroxide.

5. A pharmaceutical composition for use according to any preceding claim, selected from the group comprising;-
a composition containing 5-5,000 mg iodide, prepared at a ratio of between 0.2 : 1 and 3 : 1 by weight of iodide to hydrogen peroxide;
a composition containing 5-5,000 mg iodide, prepared at a preferable ratio of between 0.38 : 1 and 1.52 : 1 by weight of iodide to hydrogen peroxide;
a composition containing 5-5,000 mg iodide, prepared at a more preferable ratio of 0.76 : 1 by weight of iodide to hydrogen peroxide; and
a composition containing 5-5,000 mg iodide, prepared at a ratio of between 0.2 : 1 and 1 : 1 by weight of iodide to sodium percarbonate.

6. A pharmaceutical composition for use according to any preceding claim, wherein the source of iodide may be sodium iodide or potassium iodide, lithium iodide, caesium iodide, hydrogen iodide, rhodium iodide, or a slow-releasing form of iodide, such as iodates.

7. A pharmaceutical composition for use according to any preceding claim, wherein the source of hydrogen peroxide is hydrogen peroxide, sodium peroxide, lithium peroxide or peroxide releasing citric acid or Vitamin C, peroxide salts including barium oxide, sodium perborate, an hydrogen peroxide-urea adduct, oxygen releasing pseudo peroxides including superoxides, dioygenals, ozones, and ozonides, organic peroxides including peroxy acids, acyl halides, aliphatic peroxides, or a peroxide-releasing percarbonate such as sodium or potassium percarbonate, or a slow-releasing form of a peroxide-releasing percarbonate.

8. A composition for use as claimed in any preceding claim further comprising lactoferrin or a glucocorticoid, suitably wherein the glucocorticoid is predinisolone prednisone, or hydrocortisone.

9. The composition for use of any preceding claim, wherein the composition is adapted for concurrent, or sequential, delivery of the peroxide and iodide components; and/or for delivery by means of an intra-mammary device.

10. The composition for use of any preceding claim, wherein the pharmaceutically effective carrier is water, saline, an emulsion, a gel or a hydrogel.

11. A pharmaceutical composition for use according to any preceding claim, where hydrogen peroxide and iodide are provided in a concentrated form in a medical device, suitable for production of a working solution containing between 10-5,000 mg/L hydrogen peroxide and 7-3,800 mg/L iodide.

## Patentansprüche

1. Pharmazeutische Zusammensetzung zur Verwendung bei einem Verfahren zur Behandlung oder Prävention von Mastitis bei Wiederkäuern, wobei die Zusammensetzung Iodid (I-) und eine Quelle von Wasserstoffperoxid zusammen mit einem pharmazeutisch wirksamen Träger oder Verdünnungsmittel umfasst; wobei die pharmazeutische Zusammensetzung kein Peroxidase-Enzym enthält.

2. Pharmazeutisch Zusammensetzung zur Verwendung nach Anspruch 1, wobei die Konzentration von Wasserstoffperoxid weniger als 1 %, bezogen auf Gewicht/Volumen oder Gewicht/Gewicht, beträgt.

3. Pharmazeutische Zusammensetzung zur Verwendung nach einem der vorhergehenden Ansprüche, umfassend ein Gewichtsverhältnis von Iodid zu Wasserstoffperoxid von 0,2:1 bis 3:1.

4. Pharmazeutische Zusammensetzung zur Verwendung nach einem der vorhergehenden Ansprüche, umfassend ein Gewichtsverhältnis von Iodid zu Wasserstoffperoxid von 0,38:1 bis 1,52:1.

5. Pharmazeutische Zusammensetzung zur Verwendung nach einem der vorhergehenden Ansprüche, ausgewählt aus der Gruppe bestehend aus:
einer Zusammensetzung, die 5-5000 mg Iodid enthält, hergestellt in einem Gewichtsverhältnis von Iodid zu Wasserstoffperoxid zwischen 0,2:1 und 3:1;
einer Zusammensetzung, die 5-5000 mg Iodid enthält, hergestellt in einem bevorzugten Gewichtsverhältnis von Iodid zu Wasserstoffperoxid zwischen 0,38:1 und 1,52:1;
einer Zusammensetzung, die 5-5000 mg Iodid enthält, hergestellt in einem weiter bevorzugten Gewichtsverhältnis von Iodid zu Wasserstoffperoxid von 0,76:1;
einer Zusammensetzung, die 5-5000 mg Iodid enthält, hergestellt in einem Gewichtsverhältnis von Iodid zu Natriumpercarbonat zwischen 0,2:1 und 1:1.

6. Pharmazeutische Zusammensetzung zur Verwendung nach einem der vorhergehenden Ansprüche, wobei es sich bei der Quelle von Iodid um Natriumiodid oder Kaliumiodid, Lithiumiodid, Caesiumiodid, Iodwasserstoff, Rhodiumiodid oder eine langsam freigesetzte Form von Iodidid, wie Iodidate, handeln kann.

7. Pharmazeutische Zusammensetzung zur Verwendung nach einem der vorhergehenden Ansprüche, wobei es sich bei der Quelle von Wasserstoffperoxid um Wasserstoffperoxid, Natriumperoxid, Lithiumperoxid oder Peroxid freisetzende Citronensäure oder Peroxid freisetzendes Vitamin C, Peroxidsalze einschließlich Bariumoxid, Natriumperborat, ein Wasserstoffperoxid-Harnstoff-Addukt, Sauerstoff freisetzende Pseudoperoxide einschließlich Superoxiden, Dioygenalen, Ozonen und Ozoniden, organische Peroxide einschließlich Peroxysäuren, Acylhalogeniden, aliphatischen Peroxiden, oder ein Peroxid freisetzendes Percarbonat wie Natrium- oder Kaliumpercarbonat oder eine langsam freigesetzte Form eines Peroxid freisetzenden Percarbonats handelt.

8. Zusammensetzung zur Verwendung nach einem der vorhergehenden Ansprüche, ferner umfassend Lactoferrin oder ein Glucocorticoid, wobei es sich bei dem Glucocorticoid geeigneterweise um Prednisolon, Prednison oder Hydrocortison handelt.

9. Zusammensetzung zur Verwendung nach einem der vorhergehenden Ansprüche, wobei die Zusammensetzung zur gleichzeitigen oder aufeinanderfolgenden Zuführung der Peroxid- und Iodidid-Komponenten und/oder zur Zuführung mit Hilfe einer intramammären Vorrichtung ausgeführt ist.

10. Zusammensetzung zur Verwendung nach einem der vorhergehenden Ansprüche, wobei es sich bei dem pharmazeutisch wirksamen Träger um Wasser, Kochsalzlösung, eine Emulsion, ein Gel oder ein Hydrogel handelt.

11. Pharmazeutische Zusammensetzung zur Verwendung nach einem der vorhergehenden Ansprüche, wobei Wasserstoffperoxid und Iodidid in konzentrierter Form in einer medizinischen Vorrichtung, die für die Herstellung einer Arbeitslösung, die zwischen 10-5000 mg/l Wasserstoffperoxid und 7-3800 mg/l Iodid enthält, geeignet ist, bereitgestellt sind.

## Revendications

1. Composition pharmaceutique pour utilisation dans un procédé de traitement ou de prévention de la mammite chez des ruminants, la composition comprenant de l'iodure (I-) et une source de peroxyde d'hydrogène, conjointement avec un véhicule ou diluant pharmaceutiquement efficace ; la composition pharmaceutique ne comprenant pas une enzyme peroxydase.

2. Composition pharmaceutique pour utilisation selon la revendication 1, dans laquelle la concentration de peroxyde d'hydrogène est inférieure à 1 % sur une base en poids/volume ou poids/poids.

3. Composition pharmaceutique pour utilisation selon l'une quelconque des revendications précédentes comprenant un rapport de 0,2:1 à 3:1 en poids de l'iodure au peroxyde d'hydrogène.

4. Composition pharmaceutique pour utilisation selon l'une quelconque des revendications précédentes comprenant un rapport de 0,38:1 à 1,52:1 en poids de l'iodure au peroxyde d'hydrogène.

5. Composition pharmaceutique pour utilisation selon l'une quelconque des revendications précédentes, choisie dans le groupe comprenant :
une composition contenant 5 à 5 000 mg d'iodure, préparée à un rapport compris entre 0,2:1 et 3:1 en poids de l'iodure au peroxyde d'hydrogène ;
une composition contenant 5 à 5 000 mg d'iodure, préparée à un rapport préférable compris entre 0,38:1 et 1,52:1 en poids de l'iodure au peroxyde d'hydrogène ;
une composition contenant 5 à 5 000 mg d'iodure, préparée à un rapport plus préférable de 0,76:1 en poids de l'iodure au peroxyde d'hydrogène ; et
une composition contenant 5 à 5 000 mg d'iodure, préparée à un rapport compris entre 0,2:1 et 1:1 en poids de l'iodure au percarbonate de sodium.

6. Composition pharmaceutique pour utilisation selon l'une quelconque des revendications précédentes, dans laquelle la source d'iodure peut être l'iodure de sodium ou l'iodure de potassium, l'iodure de lithium, l'iodure de césium, l'iodure d'hydrogène, l'iodure de rhodium, ou une forme à libération lente d'iodure, telle que des iodates.

7. Composition pharmaceutique pour utilisation selon l'une quelconque des revendications précédentes, dans laquelle la source de peroxyde d'hydrogène est le peroxyde d'hydrogène, le peroxyde de sodium, le peroxyde de lithium ou de l'acide citrique ou de la vitamine C libérant du peroxyde, des sels de peroxyde comprenant l'oxyde de baryum, le perborate de sodium, un adduit peroxyde d'hydrogène-urée, des pseudoperoxydes libérant de l'oxygène comprenant des superoxydes, des dioxygénats, des ozones et des ozonides, des peroxydes organiques comprenant des peroxyacides, des halogénures d'acyle, des peroxydes aliphatiques, ou un percarbonate libérant un peroxyde tel que le percarbonate de sodium ou potassium, ou une forme à libération lente d'un percarbonate libérant un peroxyde.

8. Composition pour utilisation selon l'une quelconque des revendications précédentes comprenant en outre de la lactoferrine ou un glucocorticoïde, de manière appropriée dans laquelle le glucocorticoïde est la prednisolone, la prednisone ou l'hydrocortisone.

9. Composition pour utilisation selon l'une quelconque des revendications précédentes, la composition étant adaptée pour administration simultanée ou séquentielle des composants de peroxyde et d'iodure ; et/ou pour administration au moyen d'un dispositif intramammaire.

10. Composition pour utilisation selon l'une quelconque des revendications précédentes, dans laquelle le véhicule pharmaceutiquement efficace est l'eau, une solution saline, une émulsion, un gel ou un hydrogel.

11. Composition pharmaceutique pour utilisation selon l'une quelconque des revendications précédentes, dans laquelle le peroxyde d'hydrogène et l'iodure sont fournis sous une forme concentrée dans un dispositif médical, adaptée pour la production d'une solution de travail contenant entre 10 et 5 000 mg/l de peroxyde d'hydrogène et 7 à 3 800 mg/l d'iodure.
